# FASCICULE DE BREVET EUROPEEN

(11) **EP 2 785 676 B1**
(45) Date de publication et mention de la délivrance du brevet: **28.10.2015**
(21) Numéro de dépôt: 12808430.8
(22) Date de dépôt: 22.11.2012
(51) Int. Cl.: C07C 253/22, C07C 253/30, C07C 255/19, C07C 255/07, C07C 255/03, C07C 255/17

(54) **PROCEDE DE COUPURE DE CHAINES GRASSES INSATUREES**
VERFAHREN ZUM SPALTEN VON UNGESÄTTIGTEN FETTSÄUREKETTEN
METHOD FOR CLEAVING UNSATURATED FATTY CHAINS

(30) Priorité: 01.12.2011 FR 1161035
(43) Date de publication de la demande: 08.10.2014
(73) Titulaire: Arkema France, 92700 Colombes (FR)
(72) Inventeur: BRANDHORST, Markus, F-69007 Lyon (FR); DUBOIS, Jean-Luc, F-69390 Millery (FR)
(74) Mandataire: Schaefer, Anne-Sophie
(86) Numéro de dépôt international: PCT/FR2012/052686
(87) Numéro de publication internationale: WO 2013/079849

(56) Documents cités:
- WO-A1-96/39373
- GB-A- 741 739

## Description

Le travail qui a conduit à cette invention a reçu un financement de la part de l'Union Européenne dans le cadre du 7ième Programme Cadre (FP7/2007-2013) sous le numéro de projet N°241718 EUROBIOREF.

### DOMAINE DE L'INVENTION

La présente invention se rapporte à un procédé de coupure de chaînes grasses insaturées ainsi qu'à l'utilisation d'un tel procédé pour la synthèse d'aminoacides, de dinitriles, de diamines, de diacides de polyesters et/ou de polyamides.

L'invention vise notamment un procédé de synthèse d'acides ω-fonctionnalisés de formule R-(CH₂)ₙ-COOH ou R-(CH₂)ₙ(CH=CH)ₘ-COOH dans laquelle R représente COOH ou NH₂CH₂ comprenant une étape de coupure oxydante d'un dérivé insaturé d'acide gras, encore appelé « dérivé d'acide gras comportant au moins une insaturation ».

Par « dérivé insaturé d'acide gras » ou « dérivé d'acide gras comportant au moins une insaturation », on entend un composé insaturé cis ou trans comportant entre 7 et 24 atomes de carbone choisi parmi un acide gras insaturé -qu'il soit sous forme acide, ester simple ou « complexe », tel que triglycéride ou ester d'acide gras et d'alcool gras (cire végétale)- ou un nitrile gras insaturé, ledit dérivé d'acide gras étant obtenu le cas échéant à partir d'un acide gras hydroxylé, insaturé ou non.

Par « acide gras hydroxylé », on entend un acide gras comportant au moins une fonction hydroxyle, comportant entre 7 et 24 atomes de carbone, insaturé ou non -qu'il soit sous forme acide, ester simple ou « complexe », tel que triglycéride ou ester d'acide gras et d'alcool gras (cire végétale ou estolide)-.

Ainsi, lorsque ledit dérivé insaturé d'acide gras est un acide, l'étape de coupure oxydante conduit à un diacide ω-fonctionnalisé de formule HOOC-(CH₂)ₙ-COOH ou HOOC-(CH₂)ₙ(CH=CH)ₘ-COOH.

Lorsque ledit dérivé insaturé d'acide gras est un nitrile, l'étape de coupure oxydante conduit à la formation d'un nitrile-acide gras, qui, après hydrogénation de sa fonction nitrile et des éventuelles insaturations restantes, conduit à un acide ω-fonctionnalisé de formule NH₂CH₂-(CH₂)ₙ-COOH ou NH₂CH₂-(CH₂)ₙ₊₂ₘ-COOH.

Les nitrile-acides gras de formule générale NC-(CH₂)ₙ-COOH ou de formule NC(CH₂)ₙ(CH=CH)ₘCOOH (formule brute Cₙ₊₂ₘ₊₂H₂ₙ₊₂ₘ₊ᵢNO₂) dans le cas où la coupure est faite sur un nitrile gras polyinsaturé, appelés par la suite « héminitriles de diacides » ou plus simplement « héminitriles », sont des composés intermédiaires utilisables dans la synthèse de toute une gamme de composés « gras » tels que les ω-aminoacides, les α-ω-dinitriles, les α-ω-diamines, les α-ω-diacides. On entend par « nitrile acide gras » des composés linéaires ayant de 6 à 15 atomes de carbone.

### ARRIERE-PLAN TECHNIQUE

La coupure oxydante de chaînes grasses insaturées est une technique largement employée pour préparer des acides carboxyliques à partir de chaînes hydrocarbonées oléfiniques.

Cette méthode consiste typiquement à couper la double liaison d'un acide gras insaturé au moyen d'un oxydant fort pour passer d'un acide gras insaturé à longue chaîne à deux molécules saturées grasses à chaîne réduite, l'une α-ω bifonctionnelle et l'autre monofonctionnelle.

Un problème majeur rencontré lors de la mise en oeuvre de cette méthode réside dans la forte viscosité du milieu qui empêche un bon transfert de matière. Pour y remédier, les procédés de l'état de l'art sont le plus souvent mis en oeuvre en deux étapes et/ou en milieu solvant organique.

Dans les procédés en deux étapes, la première étape consiste en une oxydation de la double liaison pour former un diol vicinal. La deuxième étape consiste en la rupture de la liaison carbone-carbone entre les deux fonctions hydroxyles pour obtenir lesdites molécules saturées grasses à chaîne réduite.

On peut citer le document WO2008/138892 qui enseigne un procédé de fabrication d'acides monocarboxyliques saturés et de triglycérides d'acides carboxyliques saturés présentant au moins une fonction acide, à partir d'huiles végétales non modifiées contenant des triglycérides d'acides gras. Ce procédé comprend deux étapes distinctes : la première étape consiste à faire réagir les triglycérides d'acides gras avec un agent oxydant en présence d'un catalyseur pour obtenir un diol vicinal. La deuxième étape consiste à faire réagir le diol vicinal avec de l'oxygène en présence d'un catalyseur d'oxydation de la réaction de transformation des deux fonctions hydroxyles en fonctions acide carboxylique.

Un tel procédé en deux étapes n'est cependant pas optimal pour être mis en oeuvre à échelle industrielle. D'une part, l'emploi de deux catalyseurs différents est coûteux. D'autre part, il est contraignant d'effectuer deux étapes séparées, notamment parce qu'il est nécessaire d'effectuer un transfert de réacteur.

WO2007/039481 et WO94/10122 enseignent également des procédés de coupure oxydante de chaînes grasses insaturées en deux étapes mettant en oeuvre un catalyseur distinct dans chaque étape. Les composés de départ sont des acides gras insaturés ou leurs dérivés, par exemple leurs esters.

Dans les procédés mis en oeuvre en milieu solvant organique, il n'est possible d'utiliser qu'une faible teneur en peroxyde d'hydrogène (généralement inférieure ou égale à 30 % en poids relativement au poids total de la phase liquide) car l'utilisation simultanée d'oxygène et d'un solvant organique léger tel qu'un alcool risque de conduire à la formation d'un mélange gazeux explosif du solvant organique et de l'oxygène.

On peut citer le document US5939572 qui enseigne un procédé de préparation d'acide carboxylique comprenant une étape de mise en contact d'un composé oléfinique ou d'un dialcool vicinal avec de l'oxygène en présence d'un solvant protique polaire, d'un catalyseur inorganique oxyde (choisi parmi les oxydes de tungstène, molybdène, niobium, vanadium, tantale, titane, yttrium) et d'un peroxydant tel que le peroxyde d'hydrogène ou un acide peralcanoïque.

On peut également citer la demande WO96/39376 qui décrit la co-injection d'une solution diluée de H₂O₂ et d'oxygène moléculaire en présence de différents catalyseurs en milieu solvant organique.

Outre les problèmes de sécurité mentionnés, l'emploi de solvants est susceptible de poser des difficultés sur le plan environnemental, notamment pour se conformer aux réglementations en matière de limitation de composés organiques volatiles (COV), ou encore en termes de recyclage des rejets.

Il existe donc un besoin pour un procédé de coupure de chaînes grasses insaturées obviant, au moins partiellement, aux inconvénients précités. Notamment, il est souhaitable de disposer d'un procédé permettant d'obtenir tant des héminitriles que des diacides. Il est souhaitable de disposer d'un procédé n'utilisant pas de solvant organique. Il est souhaitable de disposer d'un procédé ayant un bon rendement en produits de coupure. Il est souhaitable de disposer d'un procédé limitant le taux de produits secondaires de la réaction, notamment ceux du type oxo-nitriles et aldéhydes, donc d'augmenter la sélectivité de la réaction. Enfin, il est souhaitable de disposer d'un procédé limitant la quantité de réactifs nécessaires (notamment l'eau oxygénée) et/ou d'effluents générés.

### RESUME DE L'INVENTION

Aussi la présente invention a t'elle pour objet un procédé de coupure de chaînes grasses insaturées, comprenant une étape de coupure oxydante dans laquelle on fait réagir en phase liquide au moins un dérivé d'acide gras comportant au moins une insaturation (ci-après dérivé insaturé d'acide gras) avec du peroxyde d'hydrogène en présence d'un catalyseur d'activation de la réaction de coupure oxydante et d'oxygène moléculaire et en l'absence de solvant organique.

Dans une première variante du procédé selon l'invention, le dérivé insaturé d'acide gras est choisi parmi : un acide gras, un ester d'acide gras, un triglycéride, un ester d'acide gras et d'alcool gras ou leurs mélanges, de sorte à obtenir à l'issue de ladite étape de coupure oxydante au moins un diacide.

Dans une deuxième variante du procédé selon l'invention, le dérivé insaturé d'acide gras est un nitrile gras répondant à la formule :

R₁-CH=CH-[(CH₂)_{q}-CH=CH]ₘ-(CH₂)ᵣ-CN

où R₁ est H ou un radical alkyle comportant de 1 à 11 atomes de carbone comportant le cas échéant une fonction hydroxyle et/ou une fonction nitrile terminale,
q, m et r sont des indices entiers tels que q =0 ou 1, 0≤ m≤ 2 et 4 ≤ r≤ 13,
ou leurs mélanges,
de sorte à obtenir à l'issue de ladite étape de coupure oxydante au moins un nitrile-acide de formule :

   HOOC-R'-CN

   où R' est un radical alkyle comportant de 4 à 13 atomes de carbone ou un radical alkylène comportant de 4 à 13 atomes de carbone et de 0 à 2 insaturations (bornes incluses).

Dans le cas ou R₁ comporte une fonction nitrile terminale, de préférence m=0, c'est-à dire que le nitrile gras est de préférence monoinsaturé.

Dans le cas ou R₁ comporte une fonction nitrile terminale, la molécule du nitrile gras est préférentiellement symétrique, ce qui permet d'obtenir deux produits identiques à l'issue de l'étape de coupure oxydante.

L'invention a aussi pour objet l'utilisation dudit procédé de coupure de chaînes grasses insaturées pour la synthèse d'aminoacides, de dinitriles, de diamines, de diacides de polyesters et/ou de polyamides.

Par « nitrile gras comportant au moins une insaturation» au sens de l'invention (ci-après nitrile gras insaturé), on entend une chaîne grasse porteuse de une voire deux fonctions nitrile, mono ou polyinsaturée, comportant de préférence entre 7 et 24 atomes de carbone par molécule.

Par « coupure oxydante » on entend la réaction selon laquelle au moins une insaturation du dérivé insaturé d'acide gras est oxydée et coupée pour obtenir deux molécules porteuses d'une fonction carbonyle, qu'elle soit acide carboxylique ou aldéhyde, sur chacun des atomes de carbone initialement reliés par ladite insaturation. De préférence, la réaction de coupure oxydante est mise en oeuvre de sorte à obtenir les molécules porteuses d'une fonction acide, mais les molécules porteuses d'une fonction aldéhyde peuvent aussi être obtenues comme coproduits et/ou intermédiaires.

Par « catalyseur d'activation de la réaction de coupure oxydante » on entend un catalyseur capable de catalyser ladite réaction de coupure oxydante.

L'étape de coupure oxydante est mise en oeuvre en l'absence de solvant organique. Par « solvant » au sens de l'invention, on entend une substance chimique capable de dissoudre en son sein des composés normalement immiscibles entre eux de sorte à permettre leur coexistence au sein d'une seule et même phase. Ainsi dans le procédé selon l'invention, la phase liquide où a lieu la réaction demeure biphasique, renfermant une phase aqueuse comprenant le peroxyde d'hydrogène et le catalyseur, et une phase organique comprenant le dérivé insaturé d'acide gras. L'oxygène et le catalyseur se répartissent dans les deux phases en fonction des coefficients de partage.

Par « phase liquide », il faut comprendre le milieu liquide constitué de la phase aqueuse et de la phase organique.

Le procédé selon l'invention présente de nombreux avantages. Il peut être mis en oeuvre aussi bien sur des acides gras insaturés (simples ou complexes) que des nitriles gras insaturés, éventuellement obtenus de façon simple à partir d'acides gras hydroxylés, insaturés ou non.

Le procédé selon l'invention permet ainsi obtenir tant des diacides que des héminitriles, à partir desquels on pourra synthétiser des aminoacides pour fabriquer le cas échéant des polyamides, des dinitriles pour fabriquer le cas échéant des diamines, ou encore des diacides pour fabriquer le cas échéant des polyamides ou des polyesters.

De façon surprenante, les inventeurs ont découvert que l'introduction d'oxygène moléculaire (O₂) dans un milieu réactionnel liquide biphasique pour opérer une réaction de coupure oxydante sur un dérivé d'acide gras à l'aide de peroxyde d'hydrogène activé améliore sensiblement le rendement en produits de coupure, avantageusement en héminitriles ou en diacides, selon l'une ou l'autre des variantes du procédé.

Par ailleurs, le fait d'effectuer la réaction de coupure oxydante en une seule étape simplifie notablement la mise en oeuvre du procédé à l'échelle industrielle, comparativement aux procédés connus.

Le procédé selon l'invention présente en outre l'avantage de diminuer le taux de produits secondaires de la réaction de coupure oxydante, tels que oxo-nitriles et aldéhydes.

De façon avantageuse, le procédé selon l'invention est mis en oeuvre en l'absence de solvant organique, ce qui limite le risque d'explosion ainsi que la quantité de COV et d'effluents générés.

Le procédé selon l'invention est aussi efficace pour limiter la consommation d'eau oxygénée.

### DESCRIPTION DETAILLEE

L'invention va maintenant être décrite plus en détail et de façon non limitative dans la description qui suit.

Lorsqu'il est fait référence à des intervalles, les expressions du type « allant de...à » incluent les bornes de l'intervalle. Inversement, les expressions du type « compris entre...et... » excluent les bornes de l'intervalle.

Sauf mention explicite, les pourcentages exprimés sont des pourcentages massiques.

Sauf mention contraire, les paramètres auxquels il est fait référence sont mesurés à pression atmosphérique.

### Obtention du dérive insaturé d'acide gras

Selon un mode de réalisation de l'invention, le dérivé insaturé d'acide gras comporte une, deux, voire trois insaturations. De façon préférée, il comporte une seule insaturation. Ceci permet de limiter la quantité de peroxyde d'hydrogène consommée lors de la réaction de coupure oxydante.

Selon un mode de réalisation de l'invention, le dérivé insaturé d'acide gras est d'origine naturelle, c'est-à-dire d'origine végétale (ce qui comprend les algues) ou animale.

Selon la première variante du procédé de l'invention, le dérivé insaturé d'acide gras est choisi parmi : un acide gras, un ester d'acide gras, un triglycéride, un ester d'acide gras et d'alcool gras ou leurs mélanges, de sorte à obtenir à l'issue de ladite étape de coupure oxydante au moins un diacide. L'étape de coupure oxydante peut comprendre, le cas échéant, une hydrolyse du composé bifonctionnel issu de la réaction de coupure oxydante en tant que telle pour obtenir le diacide (c'est par exemple le cas d'un ester-acide obtenu par coupure oxydante d'un ester d'acide gras).

Comme acide gras insaturé convenant plus particulièrement à la mise en oeuvre de l'invention, on peut citer :
- l'acide pétrosélénique (acide cis-6-octadécénoïque), son dérivé l'acide 6-hepténoïque obtenu par éthénolyse, l'acide α-linolénique (6-9-12-octadécatriénoïque), ces acides pouvant être obtenus à partir de la coriandre par exemple ;
- l'acide cis-8-eicosénoïque, l'acide cis-5,8,11,14- eicosatriénoïque (acide arachidonique), l'acide ricinoléique qui donne après déshydratation l'acide 8,10-octadécadiènoïque conjugué ;
- l'acide caproléique (cis-9-décénoïque), l'acide palmitoléique (cis-9-hexadécénoïque), l'acide myristoléique (cis-9-tétradécénoïque), l'acide oléique (cis-9-octadécénoïque), l'acide 9-décénoique obtenu par éthénolyse d'un acide oléique par exemple, l'acide élaidique (trans-9-octadécénoïque), l'acide ricinoléique (12-hydroxy-cis-9-octadécénoïque), l'acide gadoléique (cis-9-eicosénoïque), l'acide linoléique (9-12-octadécadiénoïque), l'acide ruménique (9-11-octadécadiénoïque), l'acide linoléique conjugué (9-11-octadécadiénoïque), ces acides pouvant être obtenus à partir de tournesol, colza, ricin, olive, soja, palmier, lin, avocat, argousier, coriandre, céleri, aneth, carotte, fenouil, Limnanthes (meadowfoam) ;
- l'acide linoléique conjugué 10-12 (10-12-octadécadiénoïque), l'acide 10 undécylénique obtenu par craquage thermique de l'ester méthylique de l'acide ricinoléique par exemple;
- l'acide vaccénique (cis-11-octadécénoïque), l'acide gondoïque (cis-11-eicosénoïque), l'acide lesquérolique (14-hydroxy-cis-11-eicosénoïque), l'acide cétoléique (cis-11-docosénoïque), pouvant être obtenus à partir de l'huile de Lesquerella (lesquérolique), de l'huile de Camelina sativa (gondoïque), de l'huile d'une plante de la famille des sapindaceae, de la graisse de poisson, des huiles de microalgues (cétoléique), de l'acide linoléique conjugué (9-11-octadécadiénoïque), lui-même obtenu par exemple par déshydratation de l'acide ricinoléique ;
- l'acide 12-octadécénoïque (cis ou trans) obtenu par exemple par déshydratation de l'acide 12-hydroxystéarique, l'acide linoléique conjugué 10-12 (10-12-octadécadiénoïque), l'acide 12-tridécénoïque obtenu par exemple par craquage thermique de l'ester (méthylique notamment) de l'acide lesquérolique ;
- l'acide érucique (cis-13-docosénoïque) et brassidique (trans-13-docosénoïque) qui peuvent par exemple être obtenus à partir du colza érucique, de la lunaire ou du crambe maritime (choux marin), l'acide 13-eicosenoïque (cis ou trans) obtenu par exemple par déshydratation de l'acide 14-hydroxyeicosanoique pouvant lui-même être obtenu par hydrogénation de l'acide lesquerolique ;
- l'acide 14-eicosenoïque (cis ou trans) obtenu par exemple par déshydratation de l'acide 14-hydroxyeicosanoïque lui-même obtenu par hydrogénation de l'acide lesquerolique ;
- l'acide nervonique (cis-15-tétracosoïque) qui peut être obtenu à partir de Malania oleifera et de lunaire (lunaria annua aussi connue sous le nom de monnaie du pape, honesty ou money plant en anglais) ;
- ou leurs isomères cis et trans,
- ou leurs mélanges.

Parmi les dérivés d'acides gras insaturés précités, on peut avoir un intérêt à sélectionner, dans l'ordre de disponibilité, un dérivé insaturé d'acide gras en δ-9, 5-13, δ-11, (c'est-à-dire avec la double liaison en position 9, 11 ou 13 par rapport à la fonction acide) du fait qu'ils sont les plus abondamment disponibles. Néanmoins, par ordre de préférence on sélectionnera les acides gras ayant une double liaison en position 11 ou plus, et de préférence en position 11 ou 12 ou conduisant à des nitriles insaturés ayant une double liaison en position 11 ou plus, et de préférence en position 11 ou 12.

De préférence, lorsque l'acide vaccénique est mis en oeuvre, celui-ci est d'origine naturelle, c'est-à-dire d'origine végétale (ce qui comprend les algues) ou animale. Dans un mode de réalisation préféré, l'invention a pour objet un procédé de coupure oxydante d'un nitrile gras insaturé obtenu à partir d'acide vaccénique d'origine naturelle, de sorte à obtenir l'héminitrile correspondant.

Les voies suivantes permettent de préparer un acide vaccénique d'origine naturelle :
- L'acide vaccénique peut être obtenu directement à partir de végétaux, en particulier par extraction, à partir de pulpe de mangue, d'argousier, d'huile d'argousier, ou de dérivés animaux tels que le beurre,
- l'acide vaccénique peut également être obtenu en modifiant génétiquement des plantes, telles que la carthame, la cameline, ou encore Arabidopsis thaliana comme cela est décrit dans l'article de Nguyen et al., Plant physiology, December 2010, Vol. 154, pp 1897-1904,
- l'acide vaccénique peut être obtenu à partir de bactéries ou levures génétiquement modifiées par exemple Escherichia Coli comme cela est décrit dans l'article de Mendoza et al., Journal of bacteriology September 1982, pp 1608-1611,
- une dernière voie pour obtenir l'acide vaccénique est la déshydratation ou respectivement l'ammoniation de l'acide 12-hydroxystéarique.

De préférence, lorsque l'acide gondoique est mis en oeuvre, celui-ci est d'origine naturelle, c'est-à-dire d'origine végétale (ce qui comprend les algues) ou animale. Dans un mode de réalisation préféré, l'invention a pour objet un procédé de coupure oxydante d'un nitrile gras insaturé obtenu à partir d'acide gondoique d'origine naturelle, de sorte à obtenir l'héminitrile correspondant.

Les voies suivantes permettent de préparer un acide gondoique d'origine naturelle :
- l'acide gondoique (cis-11-eicosenoique) peut être obtenu directement à partir de végétaux, en particulier par extraction, à partir de l'huile de Cameline (*Camelina Sativa*) qui contient plus de 15 % d'acide gondoique, d'huile de Colza riche en acide erucique, de Crambe, de Lunaire qui contiennent en général de 2 à 15 % d'acide gondoique, de *Allysum Maritimum* (teneur de 41.8 % en acide gondoique), de Selenia Grandis (teneur de 58.5 % en acide gondoique), de *Marshallia caespitose* (teneur de 43.9 % en acide gondoique),
- l'acide gondoique peut également être obtenu en modifiant génétiquement des plantes, telles que la cameline, ou encore *Arabidopsis thaliana,*
- l'acide gondoique peut être obtenu à partir de bactéries ou levures génétiquement modifiées par exemple Escherichia Coli,
- une dernière voie pour obtenir l'acide gondoique est l'hydrolyse de l'huile de Jojoba, qui est en fait une cire végétale (appelée huile parce que la cire est liquide à température ambiante). Cette cire est composée essentiellement d'esters d'acide gras à chaîne longue est d'alcools gras à chaîne longue. L'acide gondoique représente plus de 60 %, voire 70 % des acides gras présents dans la cire.

Les acides gras cités ci-dessus peuvent être isolés par des moyens connus de l'homme de l'art tels que la distillation moléculaire, y compris à court temps de trajet, la cristallisation, l'extraction liquide-liquide, la complexation à l'urée, y compris l'extraction au CO₂ supercritique, ou une combinaison de ces moyens.

Comme esters d'acide gras insaturé convenant plus particulièrement à la mise en oeuvre de l'invention, on peut utiliser les esters des acides gras précités.

Comme triglycéride insaturé convenant plus particulièrement à la mise en oeuvre de l'invention, on peut citer : une huile végétale comprenant un mélange de triglycérides d'acides gras insaturés tels que l'huile de tournesol, colza, ricin, lesquerella, cameline, olive, soja, palmier, sapindaceae en particulier d'avocat, argousier, coriandre, céleri, aneth, carotte, fenouil, mangue, Limnanthes Alba (meadowfoam) et leurs mélanges ; des micro-algues ; des graisses animales.

Comme exemple de cire insaturée convenant à la mise en oeuvre de l'invention, on peut citer l'huile de jojoba.

Selon un mode de réalisation, le procédé de l'invention comprend en outre une étape de fabrication du dérivé d'acide gras à partir d'un acide gras saturé hydroxylé pouvant être sous forme simple ou complexe (ester, triglycéride, cire). Ladite étape de fabrication comprend une déshydratation dudit acide gras saturé hydroxylé en acide gras insaturé. L'acide gras insaturé obtenu peut être directement soumis à l'étape de coupure oxydante conformément à la première variante du procédé selon l'invention. Il peut encore être soumis à une étape supplémentaire de nitrilation conduisant à un nitrile gras insaturé apte à subir l'étape de coupure oxydante, conformément à la deuxième variante du procédé selon l'invention.

Selon ce même mode de réalisation, le procédé de l'invention peut en outre comprendre en amont une étape de fabrication dudit acide gras saturé hydroxylé à partir d'un acide gras insaturé hydroxylé pouvant être sous forme simple ou complexe (ester, triglycéride, cire). Cette étape de fabrication comprend une hydrogénation de l'acide gras insaturé hydroxylé pour obtenir un acide gras saturé hydroxylé.

Comme acide gras saturé hydroxylé convenant particulièrement à la mise en oeuvre de l'invention on peut citer l'acide 12-hydroxylaurique, l'acide 14-hydroxymyristique, (tous deux obtenus par exemple par fermentation des acides laurique et myristique), l'acide 12-hydroxystéarique (obtenu par exemple par hydrogénation de l'acide ricinoléique et/ou de l'acide densipolique), l'acide 14-hydroxyeicosanoique (obtenu par exemple par hydrogénation de l'acide lesquérolique et/ou de l'acide auricolique), ou leurs mélanges.

Comme acide gras insaturé hydroxylé convenant plus particulièrement à la mise en oeuvre de l'invention, on peut citer : l'acide ricinoléique (12-hydroxy-cis-9-octadécénoïque), l'acide lesquérolique (14-hydroxy-cis-11-eicosénöique), l'acide densipolique et l'acide auricolique, ou leurs mélanges.

L'hydrogénation peut avantageusement être mise en oeuvre à une température allant de 70°C à 150°C, de préférence allant de 90°C à 130°C.

L'hydrogénation peut avantageusement être mise en oeuvre sous une pression de dihydrogène allant de 1 à 300 bar, de préférence de 5 à 50 bar.

L'hydrogénation peut être mise en oeuvre en présence d'un catalyseur d'hydrogénation homogène ou hétérogène. Ce dernier peut être choisi parmi un métal noble tel que Pt, Pd ou Rh, un métal de transition tel que Mo, W, Cr, Fe, Co, Ni, utilisé seul ou en mélange éventuellement déposé sur un support comportant par exemple un charbon actif, de l'alumine ou de la silice. De façon préférée, le catalyseur d'hydrogénation est un nickel de Raney ou un métal déposé sur charbon actif.

La déshydratation de l'acide gras saturé hydroxylé peut avantageusement être mise en oeuvre à une température allant de 200°C à 300°C.

La déshydratation peut avantageusement être effectuée en présence d'un catalyseur acide, de préférence choisi parmi l'acide sulfurique, l'acide phosphorique, l'acide sulfonique, un alkylsulfonate ou leurs mélanges.

Selon une variante de ce mode de réalisation, dans le cas ou le dérivé d'acide gras qui est mis en oeuvre lors de l'étape de coupure oxydante est un nitrile gras, il est possible de s'affranchir de l'étape de déshydration de l'acide gras saturé hydroxylé en lui faisant subir à la place une nitrilation et une déshydratation concomitante.

Ce mode de réalisation permet avantageusement l'obtention d'un nitrile très « propre », comprenant par exemple plus de 85% en poids de nitrile monoinsaturé relativement au poids des produits de la nitrilation.

La réaction de nitrilation (ou ammoniation, les deux termes étant utilisés indifféremment) des acides gras au moyen d'ammoniac est bien connue et suit le schéma réactionnel simplifié suivant :

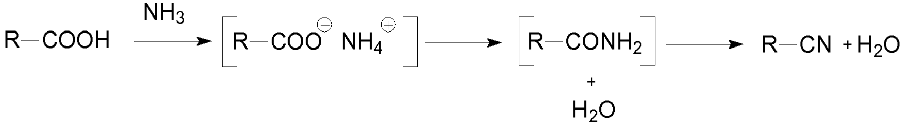

La nitrilation peut être réalisée en phase liquide (procédé batch) ou en phase gaz (procédé continu) au moyen d'ammoniac à une température généralement comprise entre 150°C et 350°C avec un catalyseur.

La nitrilation en phase liquide peut être conduite à une température comprise entre 150°C environ (première phase) et 250°-300°C (deuxième phase) avec un catalyseur qui est généralement un oxyde métallique et le plus fréquemment l'oxyde de zinc.

La nitrilation en phase gaz peut être conduite à des niveaux de température élevés et généralement sur un catalyseur constitué d'un lit fixe d'alumine dopée ou non. L'acide gras est vaporisé en présence d'une large quantité d'ammoniac dont l'excès est recyclé. Le procédé peut donc être réalisé en phase gaz avec comme catalyseur un lit fixe d'alumine dopée ou non.

Dans la nitrilation en phase gazeuse, de nombreux autres catalyseurs peuvent être utilisés et il existe une abondante littérature sur ce sujet. On peut citer par exemple le document JP200016977 qui décrit une catalyse à l'oxyde de niobium à une température de 260°C (acide stéarique), le document JP20007637 qui décrit un procédé conduit à une température allant de 180° à 350°C également avec un catalyseur de type oxyde de niobium, le brevet US6005134 qui décrit une catalyse au titane et enfin le brevet JP10195035 qui décrit une catalyse à l'oxyde de zirconium dopé au fer.

Il est également connu de GB641955 de réaliser la nitrilation en utilisant comme agent l'urée ou l'acide cyanurique. On peut aussi utiliser toute autre source d'ammoniac.

Dans le cas ou le dérivé insaturé d'acide gras qui est mis en oeuvre lors de l'étape de coupure oxydante est un nitrile gras insaturé, on préfère ceux obtenus au moins partiellement à partir d'acides ou esters gras naturels insaturés. Ainsi, selon un mode de réalisation, le procédé de l'invention comprend une étape de fabrication dudit nitrile gras à partir d'un acide ou ester gras insaturé d'origine naturelle de formule :

(R₁-CH=CH-[(CH₂)_{q}-CH=CH]ₘ-(CH₂)ᵣ-COO-)ₚ-G

où R₁ est H ou un radical alkyle comportant de 1 à 11 atomes de carbone comportant le cas échéant une fonction hydroxyle et/ou une fonction nitrile terminale,
G est H, un radical alkyle de 1 à 11 atomes de carbone ou un radical comportant 2 ou 3 atomes de carbone porteur de 1 ou 2 fonctions(s) hydroxyle(s),
q, m et r sont des indices entiers tels que q =0 ou 1, 0≤ m≤ 2 et 4 ≤ r≤ 13,
p est un indice entier tel que 1≤ p≤ 3,
ladite étape de fabrication comprenant une ammoniation de la fonction carbonyle de l'acide ou de l'ester gras insaturé d'origine naturelle en fonction nitrile.

Comme acides ou esters gras insaturés d'origine naturelle répondant à la formule ci-dessus convenant plus particulièrement à la mise en oeuvre de l'invention, on peut utiliser ceux cités précédemment.

L'ammoniation (nitrilation) peut être mise en oeuvre conformément à la méthode décrite plus haut.

Eventuellement, une étape d'hydrolyse de l'ester gras insaturé d'origine naturelle précité peut être mise en oeuvre avant l'ammoniation.

Eventuellement, une étape de déshydratation de l'acide gras hydroxylé d'origine naturelle précité peut être mise en oeuvre avant l'ammoniation.

Selon un mode de réalisation de l'invention, le dérivé insaturé d'acide gras comporte une insaturation en position 11 (δ-11) ou 12 (δ-12) ou plus par rapport à la fonction nitrile ou acide, selon l'une ou l'autre des variantes du procédé, étant de préférence choisi parmi : l'acide 11-octadécénoique, l'acide vaccénique, l'acide 11-eicosénoique, l'acide gondoïque, l'acide 12-hydroxystéarique, l'acide 14-hydroxyeicosanoique ou leurs mélanges.

De façon préférée, le dérivé insaturé d'acide gras est un acide vaccenique (C18:1, δ-11) comportant une insaturation en position 11 par rapport à la fonction acide.

De façon également préférée, le dérivé insaturé d'acide gras est un acide gondoïque (C20:1, δ-11) comportant une insaturation en position 11 par rapport à la fonction acide.

Le dérivé insaturé d'acide gras peut avantageusement être un acide lesquerollique (C20:1, δ-11, 14-OH) comportant une insaturation en position 11 par rapport à la fonction acide.

Selon un mode de réalisation de l'invention, le dérivé d'acide gras comporte plus de 18 atomes de carbone, de préférence plus de 20 atomes de carbone.

De façon préférée, le dérivé d'acide gras comporte plus de 18 atomes de carbone, encore plus préférentiellement plus de 20 atomes de carbone et comporte une insaturation en position 11, 13 ou 15 par rapport à la fonction nitrile ou acide, selon l'une ou l'autre des variantes du procédé.

### Etape de coupure oxydante

Le procédé de coupure de chaînes grasses insaturées selon l'invention comprend une étape de coupure oxydante dans laquelle on fait réagir en phase liquide au moins un dérivé d'acide gras comportant au moins une insaturation tel que ceux précités avec du peroxyde d'hydrogène en présence d'un catalyseur d'activation de la réaction de coupure oxydante et d'oxygène moléculaire et en l'absence de solvant organique.

Des dérivés d'acides gras saturés peuvent bien entendu être présents dans le milieu réactionnel en sus des dérivés d'acide gras insaturés, sans qu'ils interviennent dans la réaction de coupure oxydante. C'est notamment le cas lorsque l'on met en oeuvre une huile naturelle comme source d'acides gras insaturés dans la mesure où celle-ci comprend un mélange de divers acides saturés et insaturés.

Selon un mode de réalisation, la teneur en peroxyde d'hydrogène dans la phase liquide va de 3 % à 55 % en poids, de préférence de 4 % à 50 % en poids, voire de 5 % à 45 % en poids relativement au poids total de la phase liquide.

Selon un mode de réalisation, le peroxyde d'hydrogène est mis en oeuvre sous la forme d'une solution aqueuse de peroxyde d'hydrogène dont la concentration en peroxyde d'hydrogène va de 35% à 70% en poids, de préférence de 40% à 70% en poids, notamment de 50% à 70% en poids et plus préférentiellement de 60% à 70% en poids, relativement au poids total de ladite solution. Il est avantageux de travailler à forte concentration en peroxyde d'hydrogène pour limiter la teneur en eau de dilution dans le milieu réactionnel. Il faut cependant éviter de trop concentrer le peroxyde d'hydrogène pour limiter les risques d'explosion. La concentration en peroxyde d'hydrogène peut être contrôlée par tout moyen approprié, par exemple par dosage du H₂O₂, contrôle de l'acidité du milieu (à corréler avec la concentration en héminitrile produit), mesure de la teneur en eau, mesure de la quantité d'eau évaporée, mesure de la quantité de chaleur dégagée, mesure de la densité, mesure de la viscosité, mesure de l'indice de réfraction, spectroscopie UV, IR, Raman.

Le peroxyde d'hydrogène peut être introduit séquentiellement dans le milieu réactionnel ou, préférentiellement, en continu.

Le rapport molaire du peroxyde d'hydrogène relativement au dérivé d'acide gras peut aller de 1:1 à 20:1, notamment de 1:1 à 6:1, de préférence de 1:1 à 3:1.

Le catalyseur d'activation de la réaction de coupure oxydante (ci-après « le catalyseur ») peut notamment être choisi parmi ceux divulgués dans la demande WO 96/39373.

On pourra utiliser en particulier :
- les oxydes de tungstène ou molybdène,
- les composés capables de libérer des ions tungstate ou molybdate dans le milieu réactionnel (par exemple chlorures et sulfures)
- les oxydes de niobium, vanadium, tantale, titane ou yttrium,
- les composés métalliques capables d'être convertis en oxydes métalliques ou de libérer des ions métalliques dans le milieu réactionnel (par exemple chlorures et sulfures tels que MoS₂, MoS₃ ou VCl₃),
- les oxydes WO₂, Nb₂O₅,WO₃ ou MoO₃,
- l'acide tungstique en mélange avec l'acide molybdique, le dioxyde de titane (TiO₂), l'oxalate de titane (Ti₂(C₂O₄)₃), l'oxyde de niobium (Nb₂O₅), l'oxyde de fer (Fe₂O₃) ou éventuellement avec l'oxyde de vanadium (V₂O₅) ou le chlorure de cobalt (II),
- l'acide tungstique, le trioxyde de tungstène, l'acide molybdique, l'acide niobique, ou le pentoxyde de niobium.

De préférence, le catalyseur est choisi parmi les oxydes de tungstène, molybdène, niobium, vanadium, tantale, titane ou yttrium, leurs oxyacides inorganiques, leurs hétéropolyacides, leurs sels alcalins, les dérivés du palladium, les dérivés du manganèse, et leurs mélanges.

Comme dérivés du manganèse, on peut citer par exemple les acétates, les chlorures, les sulfates, les bromures et les nitrates. Comme dérivés du palladium, on peut citer les acétates et les sultates.

Le catalyseur est plus préférentiellement choisi parmi: le sulfate de manganèse (MnSO₄), le sulfate de palladium (PdSO₄), l'acide tungstique (H₂WO₄ le sel de sodium de l'acide tungstique (Na₂WO₄) associé à l'acide phosphorique (H₃PO₄), l'acide molybdique (H₂MoO₄), le sel de sodium de l'acide molybdique (Na₂MoO₄), des hétéro-polyacides tels que H₃[PMo₁₂O₄₀], H₄[SiMo₁₂O₄₀], H₄[SiW₁₂O₄₀], H₃[PW₁₂O₄₀], (NH₄)₁₀[H₂W₁₂O₄₂], le métavanadate de sodium (Na₃VO₄) ou le métavanadate d'ammonium ((NH₄)₃VO₄) ou un sel alcalin de ceux-ci, ou le sulfate de palladium (PdSO₄) associé à un hétéro-polyacide tel que ceux précités.

La teneur en catalyseur introduit dans la phase liquide peut aller de 0,1 % à 10 % en poids, de préférence de 0,5 % à 5 % en poids, relativement au poids total de la phase organique comprenant le dérivé d'acide gras.

Conformément à l'invention, la réaction de coupure oxydante est mise en oeuvre en présence d'oxygène moléculaire dans la phase liquide.

L'oxygène moléculaire présent lors de l'étape de coupure oxydante peut être sous forme d'oxygène moléculaire de haute pureté (teneur supérieure à 80 % mol, de préférence supérieure à 90 % mol, idéalement supérieure à 99 % mol) ou sous forme d'air ou encore sous forme d'air enrichi (c'est-à-dire à plus forte pression partielle en oxygène que dans l'air).

Selon un mode de réalisation de l'invention, l'oxygène est présent sous forme de microbulles dispersées dans la phase liquide. Ceci favorise le contact gaz-liquide et améliore la vitesse de dissolution de l'oxygène dans le liquide. Par « microbulles » on entend des bulles dont le diamètre moyen va de 1 micron à 3 mm, de préférence de 100 microns à 3 mm, de préférence de 500 microns à 1 mm. Les microbulles d'oxygène peuvent être convenablement maintenues à l'état dispersé dans le milieu réactionnel par exemple par forte agitation, ou encore par l'emploi d'une turbine auto-aspirante, d'un réacteur Loop^{®} de type Buss ChemTech, d'un microréacteur, d'un « Spinning Disk Reactor », d'un « Rotating packed bed reactor » ou d'un contacteur à film tombant.

Selon un mode de réalisation particulièrement avantageux, la réaction de coupure oxydante est mise en oeuvre en continu (le peroxyde d'hydrogène, l'oxygène et le dérivé d'acide gras sont injectés continument dans le réacteur où a lieu la réaction de coupure oxydante) et l'oxygène moléculaire est injecté à contre courant du flux de dérivé insaturé d'acide gras et de peroxyde d'hydrogène. Dans cette configuration, la réaction peut être mise en oeuvre dans un réacteur colonne, l'oxygène moléculaire étant injecté en pied de réacteur, et le nitrile gras en tête. L'eau oxygénée peut être injectée soit en tête du réacteur, soit en plusieurs points proches de la tête du réacteur. L'oxygène moléculaire, en montant dans la colonne, entraîne une partie de l'eau présente et permet de maintenir une concentration élevée en peroxyde d'hydrogène dans le réacteur. Dans cette configuration il est avantageux d'utiliser de l'air comme source d'oxygène moléculaire.

Selon un mode de réalisation, l'oxygène et le peroxyde d'hydrogène sont introduits concomitamment dans le mélange de nitrile gras et de catalyseur. Alternativement, l'oxygène peut être introduit immédiatement après ou juste avant le peroxyde d'hydrogène.

Selon un mode de réalisation de l'invention, l'oxygène est injecté dans le milieu réactionnel à une pression allant de 0,2 bar à 50 bar, notamment de 1 bar à 20 bar, de préférence de 1 à 5 bar.

Lorsque l'oxygène est sous forme d'air enrichi, notamment à plus de 80 % mol. en oxygène, la pression partielle en oxygène injecté dans le milieu réactionnel va plus préférentiellement de 5 à 20 bar.

Selon un mode de réalisation de l'invention, la teneur en oxygène moléculaire va de 100 % à 5000 % mol, de préférence de 110 % à 4000 % mol, de la stoechiométrie de la réaction de coupure oxydante. L'oxygène en excès permet avantageusement de maintenir la concentration en H₂O₂ élevée dans le réacteur, surtout s'il fonctionne en continu.

Selon un mode de réalisation de l'invention, l'eau évaporée est évacuée du milieu réactionnel en continu pendant la réaction de coupure oxydante.

Selon un mode de réalisation de l'invention, l'étape de coupure oxydante est mise en oeuvre à une température allant de 20 °C à 130 °C, notamment de 30 °C à 90 °C, de préférence de 50 °C à 90 °C. Eventuellement, la réaction peut être mise en oeuvre en au moins deux paliers successifs de température.

Lorsque la réaction est effectuée sous pression, la température peut être augmentée pour accélérer la réaction, tout en maintenant le milieu réactionnel en phase liquide.

Selon un mode de réalisation de l'invention, l'étape de coupure oxydante est mise en oeuvre pendant une durée allant de 10 minutes à 30 heures, de préférence de 30 minutes à 8 heures. Industriellement il est souhaitable de diminuer le temps de réaction, notamment en augmentant la pression et la température.

### Etapes ultérieures

Lorsque l'étape de coupure oxydante est mise en oeuvre sur un nitrile gras insaturé, il est possible que l'étape de coupure oxydante ne conduise pas directement à un héminitrile en mélange avec un acide mais à des composés intermédiaires de type aldéhyde-nitrile (aussi appelés oméga oxo-nitriles) en mélange avec un aldéhyde. Ces composés présentent l'avantage de s'oxyder très facilement au contact de l'oxygène moléculaire. On pourra ainsi mettre en oeuvre après l'étape de coupure oxydante une étape supplémentaire dite d'auto-oxydation, visant à convertir l'aldéhyde-nitrile en héminitrile et l'aldéhyde en acide correspondant.

Ainsi, selon un mode de réalisation de l'invention, le procédé comprend en outre une étape d'auto-oxydation dans laquelle le produit obtenu à l'issue de l'étape de coupure oxydante, voir l'aldéhyde-nitrile et/ou l'aldéhyde isolé(s) du reste du produit, est mis en présence d'oxygène moléculaire.

Selon un mode de réalisation de l'invention, l'étape d'auto-oxydation est mise en oeuvre en faisant buller de l'oxygène moléculaire ou un mélange gazeux contenant de l'oxygène moléculaire dans la phase liquide comprenant l'aldéhyde-nitrile et l'aldéhyde obtenue à l'issue de l'étape de coupure oxydante, éventuellement en présence du catalyseur d'activation de la réaction de coupure oxydante.

Selon un mode de réalisation particulier de l'invention, on utilise, dans l'étape d'auto-oxydation, de l'oxygène moléculaire de haute pureté, c'est-à-dire de pureté supérieure à 80 % mol, de préférence supérieure à 90 % mol, et encore plus préférablement supérieure à 99 % mol. De manière alternative, on utilise de l'air ou encore de l'air enrichi en oxygène moléculaire.

De préférence, l'étape d'auto-oxydation est mise en oeuvre sans ajout de solvant et/ou sans ajout de catalyseur d'activation de l'oxygène moléculaire. On observe une légère amélioration du rendement en aldéhyde par ajout de traces d'alcalins et/ou d'autres métaux comme catalyseur. Pour cette étape d'auto-oxydation, différents procédés sont utilisables, et notamment ceux décrits dans les documents de brevet US6680395, US6696582, US6800783, US7138544, US7799945, WO10108586, FR2769624. De préférence, lorsqu'un catalyseur alcalin est mis en oeuvre, il n'y a plus d'eau oxygénée dans le milieu. Selon un mode de réalisation particulièrement avantageux du procédé de l'invention, l'étape d'auto-oxydation est réalisée comme dans le document US6696582, sans catalyseur, en deux étapes utilisant deux paliers de températures croissantes successives d'oxydation, notamment pour maîtriser l'exothermie de la réaction. De préférence, l'auto-oxydation est réalisée dans un microréacteur, ce qui présente l'avantage d'évacuer rapidement la chaleur de réaction.

Dans le cas où l'étape d'auto-oxydation est réalisée en présence de catalyseur, on peut notamment utiliser ceux décrits dans les documents de brevet US7799945 et US7138544. Typiquement, on utilise un catalyseur alcalin (généralement sous forme de sel d'acide) et un co-catalyseur pris dans le groupe IV à XII de la classification périodique. Les alcalins améliorent les surfaces de contact gaz-liquide, et par conséquent la migration de l'oxygène dans le milieu. C'est la quantité d'oxygène dissout qui détermine la cinétique de la réaction d'auto-oxydation. L'utilisation de ces catalyseurs lors de l'auto-oxydation n'est toutefois pas essentielle. L'utilisation d'une agitation forte ou d'un microréacteur suffisent généralement pour obtenir un très bon contact gaz liquide, et donc un très bon échange de matière.

De préférence, l'étape d'auto-oxydation est mise en oeuvre sous une pression partielle d'oxygène moléculaire allant de 1 bar à 50 bar, notamment de 1 bar à 20 bar, de préférence de 1 à 5 bar. Lorsque l'oxygène moléculaire est sous forme d'air enrichi (à plus forte pression partielle que dans l'air) notamment de pureté supérieure à 80%, la pression partielle d'oxygène injecté dans le milieu réactionnel est de préférence comprise dans la gamme de 5 à 20 bar.

Avantageusement, l'oxygène est injecté en continu dans la phase liquide par bullage, de préférence sous forme d'un flux d'air ou d'oxygène moléculaire. L'oxygène injecté sous forme de microbulles favorise le contact gaz-liquide et améliore la vitesse de dissolution dans le liquide. Par «microbulles» on entend des bulles telles que définies précédemment. Toute technique de dispersion peut être utilisée et plus particulièrement celles citées précédemment relativement à l'étape de coupure oxydante.

Avantageusement, le rapport molaire de l'oxygène moléculaire relativement au nitrile-acide gras va de 3:2 à 100:2.

On arrête généralement la réaction lorsqu'on a fait passer de l'oxygène en excès, représentant plus de 100% de la stoechiométrie, de préférence plus de 110% de la stoechiométrie, de préférence plus de 120% de la stoechiométrie, voir plus de 220% de la stoechiométrie. Le rapport molaire de l'oxygène relativement au nitrile-acide gras est ainsi compris entre 100% et 5000% de la stoechiométrie et de préférence supérieur à 110%.

Avantageusement, l'auto-oxydation est mise en oeuvre à une température allant de 0°C à 100°C, de préférence de 20°C à 100°C, notamment de 30°C à 90°C, de préférence de 40°C à 80°C, éventuellement en 2 paliers consécutifs de températures croissantes.

L'invention a également pour objet l'utilisation du procédé de coupure de chaînes grasses insaturées qui vient d'être décrit pour la synthèse d'aminoacides, de dinitriles, de diamines, de diacides, de polyesters et/ou de polyamides.

Selon la première variante du procédé de l'invention, les diacides obtenus à l'issue de l'étape de coupure oxydante peuvent être utilisés dans de nombreuses applications dont la préparation polyesters, notamment par copolymérisation avec des di- ou polyols, ou la préparation de polyamides, notamment par copolymérisation avec des diamines. De préférence, les dérivés d'acide gras insaturés mis en oeuvre dans le procédé selon l'invention sont choisis de sorte à obtenir les diacides en C10, C11, C12, C13, C14, et/ou C15. On aura un intérêt particulier à choisir les dérivés d'acide gras insaturés visant à obtenir le PA11 et/ou le PA12.

Selon la deuxième variante du procédé selon l'invention, l'héminitrile obtenu à l'issue de l'étape de coupure oxydante peut être utilisé comme substrat de synthèse d'un ω-aminoacide. L'héminitrile est par exemple soumis à une réaction de réduction à l'hydrogène de sa fonction nitrile et des éventuelles insaturations C=C présentes selon le schéma réactionnel suivant :

HOOC- R'-CN +2 H₂ → HOOC- R'-CH₂NH₂

où R' désigne un radical alkyle comprenant de 4 à 13 atomes de carbone ou un radical alkylène comprenant de 4 à 13 atomes de carbone et de 0 à 2 insaturations. L'étape de réduction peut consister en une hydrogénation classique. Les catalyseurs utilisables sont nombreux mais préférentiellement on utilise un catalyseur Ru/SiC, ou les Nickel et Cobalt de Raney. Afin de favoriser la formation de l'amine primaire, on opère avec une pression partielle d'ammoniac.

L' ω-aminoacide obtenu peut à son tour être utilisé dans de nombreuses applications dont la préparation de polyamides. Selon un mode de réalisation, le procédé de l'invention comprend ainsi une étape de synthèse de polyamide par polymérisation mettant en oeuvre ledit ω-aminoacide. De préférence, les dérivés d'acide gras insaturés mis en oeuvre dans le procédé selon l'invention sont choisis de sorte à obtenir le PA9, le PA11 et/ou le PA12.

L'héminitrile obtenu à l'issue du procédé selon l'invention peut encore être utilisé comme substrat de synthèse d'un dinitrile par une réaction consécutive avec de l'ammoniac selon le schéma réactionnel suivant :

CN-R'-COOH + NH₃ → CN-R'-CN + 2 H₂O

où R' désigne un radical alkyle comprenant de 4 à 13 atomes de carbone ou un radical alkylène comprenant de 4 à 13 atomes de carbone et de 0 à 2 insaturations. Cette ammoniation de la fonction acide peut par exemple être mise en oeuvre conformément à GB741739. La réaction peut être conduite à température élevée, typiquement supérieure à 250 °C, en présence d'un catalyseur qui est généralement un oxyde métallique et le plus fréquemment l'oxyde de zinc. De préférence, les composés de départ mis en oeuvre dans le procédé selon l'invention sont choisis de sorte à obtenir les diamines en C9, C10, C11, C12 et/ou C14.

Les dinitriles obtenus peuvent encore être hydrogénés en diamines. Dans ce cas, les composés de départ mis en oeuvre dans le procédé selon l'invention sont préférentiellement choisis de sorte à obtenir les diamines en C10, C12 et/ou C14. Ces diamines peuvent à leur tour être utilisées dans de nombreuses applications dont la préparation de polyamides, notamment par réaction de ces diamines avec des diacides.

L'héminitrile obtenu à l'issue du procédé selon l'invention peut encore être utilisé comme substrat de synthèse d'un diacide par hydrolyse de sa fonction nitrile selon le schéma réactionnel suivant :

CN-R'-COOH + 2 H_{2O} → HOOC-R'-COOH + NH₃

où R' désigne un radical alkyle comprenant de 4 à 13 atomes de carbone ou un radical alkylène comprenant de 4 à 13 atomes de carbone et de 0 à 2 insaturations. L'hydrolyse se fait généralement dans des conditions acides.

Le diacide obtenu peut être utilisé dans de nombreuses applications dont la préparation de polyesters, notamment par copolymérisation avec des di- ou polyols, ou la préparation de polyamides, notamment par copolymérisation avec des diamines. De préférence, les dérivés d'acide gras insaturés mis en oeuvre dans le procédé selon l'invention sont choisis de sorte à obtenir les diacides en C10, C11, C12, C13, C14, et/ou C15.

### EXEMPLES

Les exemples suivants illustrent l'invention sans la limiter.

Pour les analyses les méthodes suivantes sont utilisées. La composition de la phase organique est analysée par chromatographie en phase gaz (GC) avec un Chromatographe HP 5890 série II à colonne HP5 munie d'un détecteur FID. La teneur en peroxyde d'hydrogène est analysée par la méthode de dosage par le permanganate CEFIC PEROXYGENS H2O2 AM7157.

### Exemple 1 : synthèse de nitrile gras insaturé

Dans un réacteur en verre préalablement séché de 0.5 L, muni d'une agitation mécanique, d'un chauffage électrique, d'un déflegmateur, d'un réfrigérant, d'un piège à carboglace, d'un système d'introduction d'ammoniac, on charge 250 g d'acide 12-hydroxystéarique. On ajoute une charge catalytique d'oxyde de zinc (0.0625 % du poids d'acide gras). Le milieu réactionnel est mis sous agitation puis chauffé jusqu'à 205 °C. Puis on introduit l'ammoniac gazeux à raison de 0.417 L/min.kg. Le milieu réactionnel est porté à 300°C. L'introduction d'ammoniac se poursuit jusqu'à ce que l'indice d'acidité du milieu réactionnel soit inférieur à 0.1 mg de KOH/g. La durée de la réaction est environ 10 h. En fin de réaction, on refroidit le milieu réactionnel à 40°C et on vidange le réacteur. Le produit est purifié par distillation puis analysé par GC. La composition du nitrile gras obtenu est indiquée dans le tableau I.

**Tableau I**

| Nitrile gras issu de l'acide 12-hydroxystéarique | % massique |
|---|---|
| C16:0 | 0,9 |
| C18:1 (δ-11 et δ-12) | 86,3 |
| C18:0 | 9,1 |
| C18:2 | 0,4 |
| C20:1 | 0,3 |
| C20:0 | 0,3 |

Le produit obtenu est relativement propre du fait de l'absence d'acide polyinsaturé dans l'acide de départ.

### Exemple 2 : coupure oxydante de chaînes de nitrile gras insaturés

### Essai 2.1 (comparatif) coupure oxydante en l'absence d'oxygène

Dans un réacteur double enveloppe de 250 cm³ comportant une agitation mécanique, 25 g du nitrile gras synthétisé dans l'exemple 1 et 250 mg d'acide tungstique (H₂WO₄ ; Merck 98%) sont introduits, puis agités et chauffés à 70°C, température maintenue par circulation d'eau thermostatée. 7g de solution aqueuse de peroxyde d'hydrogène (concentration en H₂O₂ égale à 70% en poids) est ajoutée goutte à goutte par une pompe péristaltique sur une durée de 20 minutes. Après 2h de réaction, la phase aqueuse est séparée de la phase organique et remplacée par 7g de solution fraîche de peroxyde d'hydrogène (concentration en H₂O₂ égale à 70% en poids) contenant 250 mg d'acide tungstique. Le renouvellement de la phase aqueuse est répété de façon similaire au bout de 17h, 19h, 21h et 23h, puis la réaction est arrêtée au bout de 25h. Au total, 6 ajouts de peroxyde d'hydrogène ont ainsi été effectués, pour une quantité totale de peroxyde d'hydrogène introduite de 42g. La dernière phase aqueuse est séparée de la phase organique. La phase organique restante est lavée plusieurs fois à l'eau déminéralisée jusqu'à disparition du peroxyde d'hydrogène dans l'eau de lavage puis elle est séchée sous vide et analysée par GC.

### Essai 2.2 (selon l'invention) coupure oxydante en présence d'oxygène

L'essai est opéré comme à l'essai 2.1 sauf qu'à l'issue de chaque ajout de solution aqueuse de peroxyde d'hydrogène (initialement puis au bout de 2h, 17h, 19h, 21h et 23h de réaction) on fait buller de l'air dans le milieu réactionnel liquide sous agitation à 500 tours/min. L'air est alimenté par la vanne de fond du réacteur à un débit d'air allant de 100 à 120 mL/min. Comme pour l'essai 2.1, la phase organique, une fois lavée et séchée, est analysée par GC.

Les résultats sont donnés dans le tableau II. Les concentrations en produits sont exprimées en mmol/g.

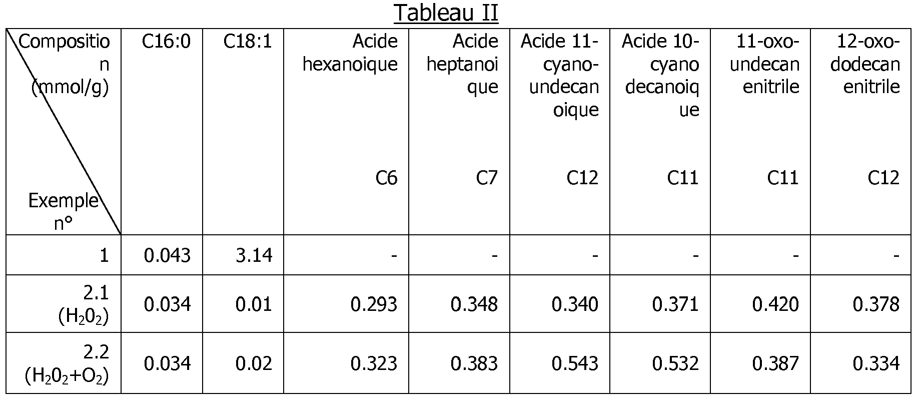

La comparaison des essais 2.1 et 2.2 montre que l'ajout concomitant de d'oxygène et de peroxyde d'hydrogène est favorable à la réaction de coupure oxydante d'un nitrile gras puisque l'on observe une augmentation de la quantité des produits de coupure comprenant une fonction acide. La quantité des héminitriles (acide 11-cyano-undecanoique (C12) et acide 10-cyano-decanoique (C11)) augmente considérablement.

### Exemple 2bis : coupure oxydante de chaînes d'acides gras insaturés

### Essai 2bis.1 (comparatif) coupure oxydante en l'absence d'oxygène

Dans un réacteur double enveloppe de 250 cm³ comportant une agitation mécanique et un réfrigérant, 32.4 g d'un mélange d'acides gras comprenant majoritairement de l'acide oléique (C18:1) (Fluka, 72.5%) et 334 mg d'acide tungstique (H₂WO₄ ; Merck 98%) sont introduits puis agités et chauffés à 90°C. Cette température est maintenue par circulation d'eau/glycol thermostatés. 36.5 g de solution aqueuse de peroxyde d'hydrogène (concentration en H₂O₂ égale à 50% en poids) est ajoutée goutte-à-goutte par une pompe péristaltique sur une durée de 5h. La réaction est arrêtée au bout de 24 h. La phase aqueuse est séparée de la phase organique. La phase organique est lavée plusieurs fois à l'eau déminéralisée jusqu'à disparition du peroxyde d'hydrogène dans l'eau de lavage puis elle est séchée sous vide et analysée par GC.

### Essai 2bis.2 (selon l'invention) coupure oxydante en présence d'oxygène

Dans un réacteur double enveloppe de 250 cm³ comportant une agitation mécanique et un réfrigérant, 32.6 g d'un mélange d'acides gras comprenant majoritairement de l'acide oléique (C18:1) (Fluka, 72.5%) et 305 mg d'acide tungstique (H₂WO₄ ; Merck 98%) sont introduits puis agités et chauffés à 90°C. Cette température est maintenue par circulation d'eau/glycol thermostatés. 36.6 g de solution aqueuse de peroxyde d'hydrogène (concentration en H₂O₂ égale à 50% en poids) est ajoutée goutte-à-goutte par une pompe péristaltique. Au bout d'une heure, un débit d'air de 400 mL/min est mis en circulation dans le mélange réactionnel *via* un tube en téflon. L'ajout goutte-à-goutte de la solution de peroxyde d'hydrogène est effectué sur une durée totale de 5h puis la réaction est arrêtée au bout de 24 h. La phase aqueuse est séparée de la phase organique. La phase organique est lavée plusieurs fois à l'eau déminéralisée jusqu'à disparition du peroxyde d'hydrogène dans l'eau de lavage puis elle est séchée sous vide et analysée par GC.

Les résultats sont donnés dans le tableau III. Les concentrations en produits sont exprimées en mmol/g.

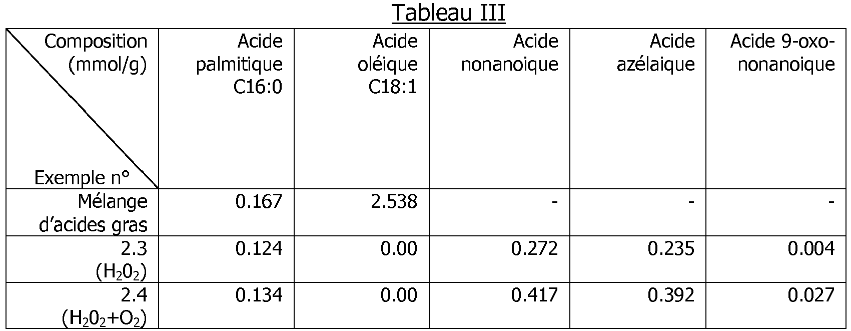

La comparaison des essais 2.3 et 2.4 montre que l'ajout concomitant de d'oxygène et de peroxyde d'hydrogène est favorable à la réaction de coupure oxydante d'un acide gras puisque l'on observe une augmentation de la quantité des produits de coupure comprenant une fonction acide. La quantité de diacide (acide azélaique) augmente considérablement.

### Essai 2bis.3 (selon l'invention) coupure oxydante en présence d'oxygène

Dans un réacteur double enveloppe de 250 cm³ comportant une agitation mécanique et un réfrigérant, 120.9 g d'un mélange d'acides gras comprenant majoritairement de l'acide eicosénoique (C20:1) (Lutacid Ee, K&H Chemicals, composition précisée dans le tableau IV) et 6.0 g d'acide tungstique (H₂WO₄ ; Merck 98%) sont introduits puis agités et chauffés à 80°C. Cette température est maintenue par circulation d'eau/glycol thermostatés. Une vanne entre le réacteur et le réfrigérant est prévue pour l'évacuation du liquide de reflux. 168.0 g de solution aqueuse de peroxyde d'hydrogène (concentration en H₂O₂ égale à 65% en poids) est ajoutée goutte-à-goutte par une pompe péristaltique sur une durée totale de 6h de la façon suivante : au bout de la première heure, un débit d'air de 400 mL/min est mis en circulation dans le mélange réactionnel à l'aide d'un tube plongeant en téflon ; entre la première et la sixième heure incluse, la vanne est ouverte pour permettre l'évacuation du liquide de reflux ; après la sixième heure, la vanne est fermée et le débit d'air est diminué à 200 mL/min. La réaction est arrêtée au bout de 24 h. La phase aqueuse est séparée de la phase organique. La phase organique est lavée plusieurs fois à l'eau déminéralisée jusqu'à disparition du peroxyde d'hydrogène dans l'eau de lavage puis elle est séchée sous vide et analysée par GC.

Les résultats sont donnés dans le tableau V. Les concentrations en produits sont exprimées en mmol/g.

**Tableau IV**

| Composition mélange d'acides gras | % massique |
|---|---|
| C18:1 | 21,38 |
| C20:1 | 46,29 |
| C20:0 | 4,29 |
| C22:1 | 12,69 |

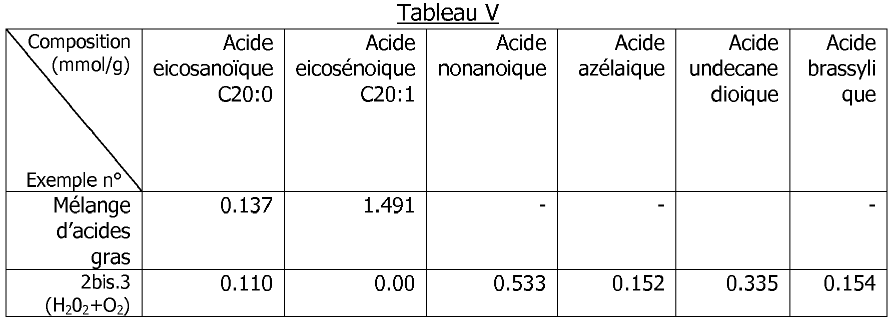

### Exemple 3 : réduction des héminitriles pour obtenir des aminoacides

On reproduit l'exemple 2.2 en poursuivant l'ajout d'air pendant 24 heures à 80 °C, en mesurant les teneurs en cyanoacides et en oxonitrile. Les cyanoacides étant solubles dans l'acide acétique, le produit de la réaction de coupure est extrait avec un mélange acide acétique/eau, puis ce solvant et les acides légers (hexanoique et heptanoique) sont évaporés. Le produit est recristallisé une fois dans l'acide acétique.

Un catalyseur Ru/SiC est introduit dans un autoclave en inox équipé d'un agitateur électromagnétique et d'une capacité de 500 ml. Une solution contenant 5 g du mélange d'acide 11-cyanoundécanoique et d'acide 10-cyanodécanoique obtenu conformément à l'invention et de solvant mixte de 140 ml de n-propanol et de 140 ml d'ammoniaque à 28 % en poids d'ammoniac est introduite dans l'autoclave. Après avoir purgé le réacteur plusieurs fois avec de l'azote, le réacteur est pressurisé à 35 bars avec de l'hydrogène. Le réacteur est ensuite chauffé à 110 °C et l'agitation et la température sont maintenues constantes pendant 1h et 30 minutes. La réaction ne consomme alors plus d'hydrogène et l'autoclave est redescendu en température jusque 70 °C, puis la pression est réduite à la pression atmosphérique et un liquide incolore est soutiré. Le solvant est ensuite évaporé à environ 60 °C sous vide, et des cristaux blancs (4 g) de mélange d'acide 11-aminoundecanoique et d'acide 12-aminododécanoique sont récupérés.

## Revendications

1. Procédé de coupure de chaînes grasses insaturées comprenant une étape de coupure oxydante dans laquelle on fait réagir en phase liquide au moins un dérivé d'acide gras comportant au moins une insaturation avec du peroxyde d'hydrogène en présence d'un catalyseur d'activation de la réaction de coupure oxydante et d'oxygène moléculaire et en l'absence de solvant organique.

2. Procédé selon la revendication précédente, dans lequel ledit dérivé d'acide gras comportant au moins une insaturation est choisi parmi : un acide gras, un ester d'acide gras, un triglycéride, un ester d'acide gras et d'alcool gras ou leurs mélanges, de sorte à obtenir à l'issue de ladite étape de coupure oxydante au moins un diacide.

3. Procédé selon la revendication 1, dans lequel ledit dérivé d'acide gras est un nitrile gras répondant à la formule :
R₁-CH=CH-[(CH₂)_{q}-CH=CH]ₘ-(CH₂)ᵣ-CN
où R₁ est H ou un radical alkyle comportant de 1 à 11 atomes de carbone comportant le cas échéant une fonction hydroxyle et/ou une fonction nitrile terminale,
q, m et r sont des indices entiers tels que q =0 ou 1, 0≤ m≤ 2 et 4 ≤ r≤ 13, ou leurs mélanges,
de sorte à obtenir à l'issue de ladite étape de coupure oxydante au moins un nitrile-acide de formule :
HOOC-R'-CN
où R' est un radical alkyle comportant de 4 à 13 atomes de carbone ou un radical alkylène comportant de 4 à 13 atomes de carbone et de 0 à 2 insaturations.

4. Procédé selon la revendication 3, comprenant en outre une étape de fabrication dudit nitrile gras à partir d'un acide ou ester gras insaturé d'origine naturelle de formule :
(R₁-CH=CH-[(CH₂)_{q}-CH=CH]ₘ-(CH₂)ᵣ-COO-)ₚ-G
où R₁ q, m et r sont tels que définis ci-dessus,
p est un indice entier tel que 1≤ p≤ 3,
G est H, un radical alkyle de 1 à 11 atomes de carbone ou un radical comportant 2 ou 3 atomes de carbone porteur de 1 ou 2 fonctions(s) hydroxyle(s),
ladite étape de fabrication comprenant une ammoniation de la fonction carbonyle de l'acide ou de l'ester gras insaturé d'origine naturelle en fonction nitrile.

5. Procédé selon la revendication 3 ou 4, comprenant en outre une hydrogénation dudit nitrile-acide conduisant à un ω-aminoacide de formule :
HOOC-R'-CH₂NH₂
où R' est un radical alkyle comprenant de 4 à 13 atomes de carbone ou un radical alkylène comportant de 4 à 13 atomes de carbone et de 0 à 2 insaturations.

6. Procédé selon la revendication 2 ou 5, comprenant en outre une étape de synthèse de polyamide par polymérisation mettant en oeuvre ledit diacide ou ledit ω-aminoacide.

7. Procédé selon l'une des revendications précédentes dans lequel lors de l'étape de coupure oxydante, l'oxygène est présent sous forme de microbulles dispersées dans la phase liquide.

8. Procédé selon l'une des revendications précédentes dans lequel le catalyseur d'activation de la réaction de coupure oxydante est choisi parmi les oxydes de tungstène, molybdène, niobium, vanadium, tantale, titane ou yttrium, leurs oxyacides inorganiques, leurs hétéropolyacides, leurs sels alcalins, les dérivés du palladium, les dérivés du manganèse, et leurs mélanges.

9. Procédé selon l'une des revendications précédentes dans lequel l'étape de coupure oxydante est mise en oeuvre sous une pression d'oxygène allant de 0,2 bar à 50 bar, notamment de 1 bar à 20 bar, de préférence de 1 à 5 bar.

10. Procédé selon l'une des revendications précédentes, dans lequel la teneur en oxygène moléculaire va de 100% à 5000%, de préférence de 110% à 4000%, de la stoechiométrie de la réaction de coupure oxydante.

11. Procédé selon l'une des revendications précédentes dans lequel la teneur en peroxyde d'hydrogène dans la phase liquide va de 3 % à 55 % en poids, de préférence de 4 % à 50 % en poids, voire de 5 % à 45 % en poids relativement au poids total de la phase liquide.

12. Procédé selon l'une des revendications précédentes dans lequel lors de l'étape de coupure oxydante le rapport molaire du peroxyde d'hydrogène relativement au dérivé d'acide gras va de 1:1 à 20:1, notamment de 1:1 à 6:1, de préférence de 1:1 à 3:1.

13. Procédé selon l'une des revendications précédentes dans lequel l'étape de coupure oxydante est mise en oeuvre à une température allant de 20°C à 130°C, notamment de 30°C à 90°C, de préférence de 50°C à 90°C.

14. Procédé selon l'une des revendications précédentes dans lequel le dérivé d'acide comporte au moins une insaturation en position 11, 12 ou plus par rapport à sa fonction nitrile ou acide, étant de préférence choisi parmi : l'acide 11-octadécénoique, l'acide vaccénique, l'acide 11-eicosénoique, l'acide gondoïque, ou leurs mélanges.

15. Utilisation du procédé selon l'une quelconque des revendications précédentes pour la synthèse d'aminoacides, de dinitriles, de diamines, de diacides, de polyesters et/ou de polyamides.

## Patentansprüche

1. Verfahren zum Spalten von ungesättigten Fettketten, umfassend einen Schritt der oxidativen Spaltung, bei dem man in der Flüssigphase mindestens ein Fettsäurederivat mit mindestens einer Ungesättigtheit in Gegenwart von einem Katalysator zur Aktivierung der Reaktion der oxidativen Spaltung und molekularem Sauerstoff und in Abwesenheit von organischem Lösungsmittel mit Wasserstoffperoxid umsetzt.

2. Verfahren nach dem vorhergehenden Anspruch, bei dem man das Fettsäurederivat mit mindestens einer Ungesättigtheit aus einer Fettsäure, einem Fettsäureester, einem Triglycerid, einem Ester von Fettsäure und Fettalkohol oder Mischungen davon auswählt, so dass man am Ende des Schritts der oxidativen Spaltung mindestens eine Disäure erhält.

3. Verfahren nach Anspruch 1, bei dem es sich bei dem Fettsäurederivat um ein Fettsäurenitril, das der folgenden Formel entspricht:
R₁-CH=CH-[(CH₂)_{q}-CH=CH]ₘ-(CH₂)ᵣ-CN
wobei R₁ für H oder einen Alkylrest mit 1 bis 11 Kohlenstoffatomen und gegebenenfalls einer Hydroxylfunktion und/oder einer endständigen Nitrilfunktion steht,
q, m und r für solche ganzzahligen Indices stehen, dass q = 0 oder 1, 0 ≤ m ≤ 2 und 4 ≤ r ≤ 13,
oder Mischungen davon handelt,
so dass man am Ende des Schritts der oxidativen Spaltung mindestens eine Nitrilsäure der Formel:
HOOC-R'-CN
erhält, wobei R' für einen Alkylrest mit 4 bis 13 Kohlenstoffatomen oder einen Alkylenrest mit 4 bis 13 Kohlenstoffatomen und 0 bis 2 Ungesättigtheiten steht.

4. Verfahren nach Anspruch 3, ferner umfassend einen Schritt der Herstellung des Fettsäurenitrils aus einer ungesättigten Fettsäure oder einem ungesättigten Fettsäureester natürlichen Ursprungs der Formel:
(R₁-CH=CH-[(CH₂)_{q}-CH=CH]ₘ-(CH₂)ᵣ-COO-)ₚ-G
wobei R₁, q, m und r wie oben definiert sind,
p für einen solchen ganzzahligen Index steht, dass 1 ≤ p ≤ 3,
G für H, einen Alkylrest mit 1 bis 11 Kohlenstoffatomen oder einen Rest mit 2 oder 3 Kohlenstoffatomen, der 1 oder 2 Hydroxylfunktion(en) trägt, steht,
wobei der Herstellungsschritt eine Ammonierung der Carbonylfunktion der ungesättigten Fettsäure bzw. des ungesättigten Fettsäureesters natürlichen Ursprungs zu einer Nitrilfunktion umfasst.

5. Verfahren nach Anspruch 3 oder 4, ferner umfassend eine Hydrierung der Nitrilsäure, die zu einer ω-Aminosäure der Formel:
HOOC-R'-CH₂NH₂
führt, wobei R' für einen Alkylrest mit 4 bis 13 Kohlenstoffatomen oder einen Alkylenrest mit 4 bis 13 Kohlenstoffatomen und 0 bis 2 Ungesättigtheiten steht.

6. Verfahren nach Anspruch 2 oder 5, ferner umfassend einen Schritt der Synthese von Polyamid durch Polymerisation unter Verwendung der Disäure oder der ω-Aminosäure.

7. Verfahren nach einem der vorhergehenden Ansprüche, bei dem bei dem Schritt der oxidativen Spaltung der Sauerstoff in Form von in der Flüssigphase dispergierten Mikrobläschen vorliegt.

8. Verfahren nach einem der vorhergehenden Ansprüche, bei dem man den Katalysator zur Aktivierung der Reaktion der oxidativen Spaltung aus den Oxiden von Wolfram, Molybdän, Niob, Vanadium, Tantal, Titan oder Yttrium, anorganischen Oxosäuren davon, Heteropolysäuren davon, Alkalisalzen davon, Palladiumderivaten, Manganderivaten und Mischungen davon auswählt.

9. Verfahren nach einem der vorhergehenden Ansprüche, bei dem man den Schritt der oxidativen Spaltung unter einem Sauerstoffdruck im Bereich von 0,2 bar bis 50 bar, insbesondere 1 bar bis 20 bar, vorzugsweise 1 bis 5 bar, durchführt.

10. Verfahren nach einem der vorhergehenden Ansprüche, bei dem der Gehalt an molekularem Sauerstoff im Bereich von 100% bis 5000%, vorzugsweise 110% bis 4000%, der Stöchiometrie der Reaktion der oxidativen Spaltung liegt.

11. Verfahren nach einem der vorhergehenden Ansprüche, bei dem der Gehalt an Wasserstoffperoxid in der Flüssigphase im Bereich von 3 bis 55 Gew.-%, vorzugsweise 4 bis 50 Gew.-% oder sogar 5 bis 45 Gew.-%, bezogen auf das Gesamtgewicht der Flüssigphase, liegt.

12. Verfahren nach einem der vorhergehenden Ansprüche, bei dem bei dem Schritt der oxidativen Spaltung das Molverhältnis von Wasserstoffperoxid zu Fettsäurederivat im Bereich von 1:1 bis 20:1, insbesondere 1:1 bis 6:1, vorzugsweise 1:1 bis 3:1, liegt.

13. Verfahren nach einem der vorhergehenden Ansprüche, bei dem man den Schritt der oxidativen Spaltung bei einer Temperatur im Bereich von 20°C bis 130°C, insbesondere 30°C bis 90°C, vorzugsweise 50°C bis 90°C, durchführt.

14. Verfahren nach einem der vorhergehenden Ansprüche, bei dem das Säurederivat mindestens eine Ungesättigtheit in Position 11, 12 oder mehr in Bezug auf seine Nitril- oder Säurefunktion umfasst und vorzugsweise aus 11-Octadecensäure, Vaccensäure, 11-Eicosensäure, Gondosäure oder Mischungen davon ausgewählt wird.

15. Verwendung des Verfahrens nach einem der vorhergehenden Ansprüche zur Synthese von Aminosäuren, Dinitrilen, Diaminen, Disäuren, Polyestern und/oder Polyamiden.

## Claims

1. A method for cleaving unsaturated fatty chains comprising a step of oxidative cleavage in which at least one fatty acid derivative having at least one unsaturation is reacted in the liquid phase with hydrogen peroxide in the presence of a catalyst for activating the reaction of oxidative cleavage and of molecular oxygen and in the absence of organic solvent.

2. The method as claimed in the preceding claim, wherein said fatty acid derivative having at least one unsaturation is selected from: a fatty acid, a fatty acid ester, a triglyceride, an ester of fatty acid and fatty alcohol or mixtures thereof, so as to obtain at least one diacid at the end of said oxidative cleavage step.

3. The method as claimed in claim 1, wherein said fatty acid derivative is a fatty nitrile corresponding to the formula:
R₁-CH=CH-[(CH₂)_{q}-CH=CH]ₘ-(CH₂)ᵣ-CN
where R₁ is H or an alkyl radical comprising from 1 to 11 carbon atoms if necessary comprising a hydroxyl function and/or a terminal nitrile function,
q, m and r are integral indices such that q =0 or 1, 0≤ m≤ 2 and 4 ≤ r≤ 13, or mixtures thereof,
so as to obtain, at the end of said oxidative cleavage step, at least one nitrile-acid of formula:
HOOC-R'-CN
where R' is an alkyl radical comprising from 4 to 13 carbon atoms or an alkylene radical comprising from 4 to 13 carbon atoms and from 0 to 2 unsaturations.

4. The method as claimed in claim 3, further comprising a step of manufacturing said fatty nitrile starting from an unsaturated fatty acid or ester of natural origin of formula:
(R₁-CH=CH-[(CH₂)_{q}-CH=CH]ₘ-(CH₂)ᵣ-COO-)ₚ-G
where R₁ q, m and r are as defined above,
p is an integral index such that 1≤ p≤ 3,
G is H, an alkyl radical with 1 to 11 carbon atoms or a radical comprising 2 or 3 carbon atoms bearing 1 or 2 hydroxyl function(s),
said manufacturing step comprising ammoniation of the carbonyl function of the unsaturated fatty acid or ester of natural origin to a nitrile function.

5. The method as claimed in claim 3 or 4, further comprising hydrogenation of said nitrile-acid leading to an ω-amino acid of formula:
HOOC-R'-CH₂NH₂
where R' is an alkyl radical comprising from 4 to 13 carbon atoms or an alkylene radical comprising from 4 to 13 carbon atoms and from 0 to 2 unsaturations.

6. The method as claimed in claim 2 or 5, further comprising a step of polyamide synthesis by polymerization using said diacid or said ω-amino acid.

7. The method as claimed in one of the preceding claims, wherein during the step of oxidative cleavage, oxygen is present in the form of microbubbles dispersed in the liquid phase.

8. The method as claimed in one of the preceding claims, wherein the catalyst for activating the reaction of oxidative cleavage is selected from the oxides of tungsten, molybdenum, niobium, vanadium, tantalum, titanium or yttrium, their inorganic oxy acids, their heteropolyacids, their alkaline salts, palladium derivatives, manganese derivatives, and mixtures thereof.

9. The method as claimed in one of the preceding claims, wherein the step of oxidative cleavage is carried out at an oxygen pressure ranging from 0.2 bar to 50 bar, notably from 1 bar to 20 bar, preferably from 1 to 5 bar.

10. The method as claimed in one of the preceding claims, wherein the content of molecular oxygen is in the range from 100 to 5000%, preferably from 110% to 4000%, of the stoichiometry of the reaction of oxidative cleavage.

11. The method as claimed in one of the preceding claims, wherein the content of hydrogen peroxide in the liquid phase is in the range from 3 to 55 wt%, preferably from 4 to 50 wt%, or even from 5 to 45 wt% relative to the total weight of the liquid phase.

12. The method as claimed in one of the preceding claims, wherein during the step of oxidative cleavage the molar ratio of hydrogen peroxide to the fatty acid derivative is in the range from 1:1 to 20:1, notably from 1:1 to 6:1, preferably from 1:1 to 3:1.

13. The method as claimed in one of the preceding claims, wherein the step of oxidative cleavage is carried out at a temperature ranging from 20°C to 130°C, notably from 30°C to 90°C, preferably from 50°C to 90°C.

14. The method as claimed in one of the preceding claims, wherein the acid derivative comprises at least one unsaturation in position 11, 12 or more relative to its nitrile or acid function, preferably being selected from: 11-octadecenoic acid, vaccenic acid, 11-eicosenoic acid, gondoic acid or mixtures thereof.

15. The use of the method as claimed in any one of the preceding claims for the synthesis of amino acids, dinitriles, diamines, diacids, polyesters and/or polyamides.
